# EUROPEAN PATENT APPLICATION

(11) **EP 2 835 105 A1**
(43) Date of publication of application: **11.02.2015**
(21) Application number: 13179418.2
(22) Date of filing: 06.08.2013
(51) Int. Cl.: A61B 17/17, A61B 17/56, A61B 19/00

(54) **Surgical guidance system**

(71) Applicant: Point Targeting AG, 5210 Windisch (CH)
(72) Inventor: Stifter, Jan, 5425 Schneisingen (CH); Hofmann, Michael, 23669 Timmendorfer Strand (DE)
(74) Representative: Parker, Andrew James

(57) **Abstract**

A surgical guidance system (1) for orthoscopic drilling is disclosed which comprises a probe (10), the end (12) of which is suitable for locating on the emergence site of a hole to be drilled through body tissue, a targeter (20) comprising one or more sensors for locating the position of the probe (10), a drilling guide (21), and a control system (30) in communication with the targeter (20) for collating the position of the probe (10) measured by the targeter (10) and providing a real-time visual representation (31) of the relationship between the direction of the drilling guide (21) and the end of the probe (12) to allow the user to align the drilling guide (21) to the end of the probe (12). The probe (21) comprises an elongate shaft (13) and one or more, preferably two or more, elements (11) located at physically separated positions along the shaft (13), and wherein further the elements (11) can be detected by the targeter (20) and are each positioned at locations removed from the end of the probe (12).

## Description

New surgical techniques are being constantly developed and improved, and in a number of situations these surgical techniques relate to correction of damage to ligaments or other aspects relating to joints and the like. In particular, for active people it is not uncommon for damage to occur in joints and ligaments of the body which need surgical techniques to properly repair and restore the normal freedom of movement to the body. Many of these techniques are desirably undertaken by means of orthoscopic techniques; generally these techniques meaning that the surgical site is not fully open or investigated during the surgical procedure, rather the surgeon uses a small camera within the cavity or joint of the patient in order to visualise what is occurring.

Many surgical techniques, for example those of transcoracoid-transclavicular drilling, preferably require the surgeon to make a bore or drill hole through a portion of the patient's body to a desired site, wherein the end of the bore hole is defined within the patient's body but not visible to the surgeon when drilling. If, for example, a surgeon is required to drill through the bone of the patient into the centre of a joint, for example to the underside of the joint cartilage, it is desirable for the patient's joint to be generally undisturbed and for the drill bore to proceed to the desired point within the joints - without the surgeon viewing the end point of the bore from the other side. That is, in many situations the surgical procedure is much improved by leaving the cartilage in an undisturbed, or minimally disturbed, state, thus meaning that the surgeon uses their skill in locating the bore and stopping the drill at the desired end point within the joint.

Know surgical guidance systems exist, for example the Smith & Nephew Trigen Sure-Shot System, wherein these systems provide a targeter which can locate a chip present or inluded in an implant within the patient. Certain surgical procedures require the introduction of an intramedullary nail into the bone of a patient., wherein this nail is to be fixed by means of screws and bolts. In order to find the fixation holes on the nail, the Smith & Nephew System introduces a chip which can be sensed by the targeting system so as to locate the bore in the intramedullary nail to allow for successful drilling by the surgeon. Location of the chip positioned within the implant in the patient's body thus allows the surgeon to make a proper drill hole into the bone of the patient and successfully find the fixation bore of the implant.

This known technique provides a mechanism of reliably locating a bore within an implant placed in the body of a patient. Such a technique does improve the accuracy of a surgeon drilling into a patient, however this requires that the patient has undergone serious surgery in order to introduce an implant within the bone. This system is not intended for use in simply drilling into the bone of a patient, and explicitly requires the use of both the implant with a chip and the targeter.

### SUMMARY OF THE INVENTION

The present disclosure relates to a guidance system which can be used by a surgeon in situations where a patient has not already undergone surgery to have an implant placed within the body. In particular, this system allows for accurate guidance of a drill bit and drill bore without the necessity of implanting a targeting chip or beacon within the body of the patient in order to guide the surgeon. The guidance system is best seen in the discussion of independent claim 1, wherein a method of properly calibrating this system is defined in independent claim 13.

The present disclosure relates to a guidance system for surgery and in particular one for improvement of drilling in an orthoscopic surgical technique. The system comprises a probe, wherein the probe is preferably utilised for highlighting the end point within the patient's body to which the drill bore should extend. As such, the probe is preferably structured such that the end point of the probe can be positioned within a patient's body. Further, the system advantageously comprises a targeter or target module, wherein the targeter is configured to be able to sense the end of the probe, and thus the end point of a drill line. The system further incorporates some form of drill guide or drill tube for providing a guidance to a drill bit when drilling into the patient's body, such that measurements made by the targeter can be used to align the drill guide to the desired end point of the bore to be drilled.

A control system may be provided which is to be used in conjunction with the targeter providing a real time visualisation of the end of the probe and the targeter. In particular, the control system may be provided to highlight the direction in which drilling would proceed through the targeter, and the end of the probe to ensure that drilling can proceed in a successful manner. The probe may preferably comprise an elongate shaft, such that the probe can be inserted into a patient's body. Along this shaft one or more elements which can be sensed by the targeter can be positioned, such that the targeter and control system may determine the location of the probe, and in particular the end of the probe to allow for the system to properly guide the drill. These elements are specifically, and preferably, located away from the probe's end.

The control system is preferably adapted such that it may make measurements or take measurements from the targeter or targeting module, and from these measurements make a determination of the end of the probe and thus the desired point for the end of the drill bore. Not only with this system sense the direction along the drilling line through the drill guide, but will also sense the distance from the drill guide to the end of the probe to allow for the correct bore to be made.

In order to allow the surgeon to properly locate the end of the probe by means of the targeter, the system may also comprise some means of generating an image for the surgeon such that the end of the probe, the direction and the end of the drill guide can be readably seen and grasped. This means that the drill guide can be properly aligned to provide a desired drilling direction to the end of the probe, and the angle of the drill guide can also be shown on the screen to the user to also allow for the surgeon to select a desired angle in which to drill, which is often very important in certain surgical procedures.

Preferably, the entire system is devised in such a manner that the visual representation of the targeter, and in particular the direction of the drill guide, and the end of the probe correlates with the real world directions of the drill guide and probe. If the surgeon is using the system wherein the onscreen or other visual representation of the system matches with the real world directions, movement of the targeter to the left will also mean that the image changes to move the visual representation of the targeter with respect to the probe to the left. It will be understood that this greatly improves the intuitive nature of the system, and leads to great improvement in the accuracy and speed of use of the system in locating the correct position of the drill guide with respect to the probe. Furthermore, in procedures where the angle of the drill guide needs to be known, the system can properly correlate the drill guide angle with the end of the probe and show this relationship on the visual representation.

A calibration routine or subsection of the control system may be provided, such that the surgeon can properly calibrate the real world orientation of the probe and store this in the system. By allowing the direction of the probe to be calibrated in use, the surgeon is not restricted in the actual direction in which the probe is positioned into the patient, and greater flexibility of the technique is given.

In order to begin the calibration routine, the control system is preferably adapted to recognise a triggering act or signal. The actual act or signal is not in any way limited, however it is preferable that this act or signal is unlikely to arise during actual surgical procedure, so as to avoid an inadvertent calibration routine rather than proper targeting of the system.

For the triggering act, it may be desirable to have the end of the targeter brought into close proximity with the end of the probe; for the surgeon to provide a certain predetermined set of motions of the targeter near the probe; to actuate a switch or other element on the probe or targeter; to move the targeter in such a manner that the probe cannot be sensed by the targeter, wherein preferably this could be done for a predetermined length of time.

It is further preferable and possible to provide extra steps in this method, such that after calibration has occurred, the control system can monitor any relative motion between the probe and the targeter and determine this to be a change in the real world orientation of the probe - rather than an actual target procedure. In allowing the continued updating of the real world orientation of the probe, the surgeon does not need to keep the orientation of the probe generally the same after calibration, and further the intuitive nature of the system and accuracy of the surgery will be improved.

A preferable structure to the probe is to comprise elements which can be seen by the targeter at a position on the probe which is not going to be positioned within the patient's body during targeting. By keeping the elements which will be sensed by the targeter away from interference of the patient's body, the accuracy and speed of the system will be improved as the patient's body will not cause interference and imaging problems in the system.

Whilst there is no real limitation on the elements which are to be sensed and positioned on the probe, these elements could be any one or more of coils, magnets, RFIDs, denser portions of the shaft, other electromagnetically discernible elements. It is also preferable that if multiple elements are provided, each of these elements is different. The difference is in no way limited, however by providing a difference which can be sensed by the targeter, the end of the probe can be determined by means of the two elements in a quicker and more accurate manner. To this end, it is preferable if the targeter is in some way an electromagnetic sensor such that the elements can be readily viewed.

A further modification in the system could be to combine the drill guide or drill tube with the targeter, so that the targeter and drill guide form a combined unit. This has the advantage that the targeter can be used to locate the end of the probe and then can at the same time properly align the drill guide for performing the desired surgery.

As is clear, a method for calibration of the above system can also be provided. In particular, the method of use of the above targeter, probe and control system, could allow for some triggering step to reset any predefined orientation of the probe in the system. Once the triggering step has been registered by the control system, the control system can provide instructions to the user via the real time visual representation, so as to instruct the user how to move one or other of the probe or targeter with respect to the other of the targeter or probe. The system may then monitor the relative motion between the probe and the targeter, and use this to determine the actual real world orientation of the probe in use. Such a method allows for the surgeon to properly program the system to understand the real world direction of the probe, and thus improve the visual representation and visual movement in correlation with the real world movement.

It is preferable that this method be provided with a continued learning phase, wherein after the calibration has occurred the system monitors all movement of the probe with relation to the targeter and uses this to update the real world orientation of the probe. In such a manner, the accuracy of the visual representation can be improved even further thus ensuring that surgery can proceed in a swift an accurate manner.

### DESCRIPTION OF THE FIGURES

- Fig. 1:: A representation showing a targeter and probe for use in guidance of a drill in a surgical technical.
- Fig. 2:: Use of the system in Fig. 1 in a shoulder joint.
- Fig. 3:: Use of the system shown in Fig. 1 for locating a drilling through a patient's bone.
- Fig. 4:: Example onscreen visual representation showing the guidance of the targeter to the end of the probe.

### DETAILED DESCRIPTION OF THE INVENTION

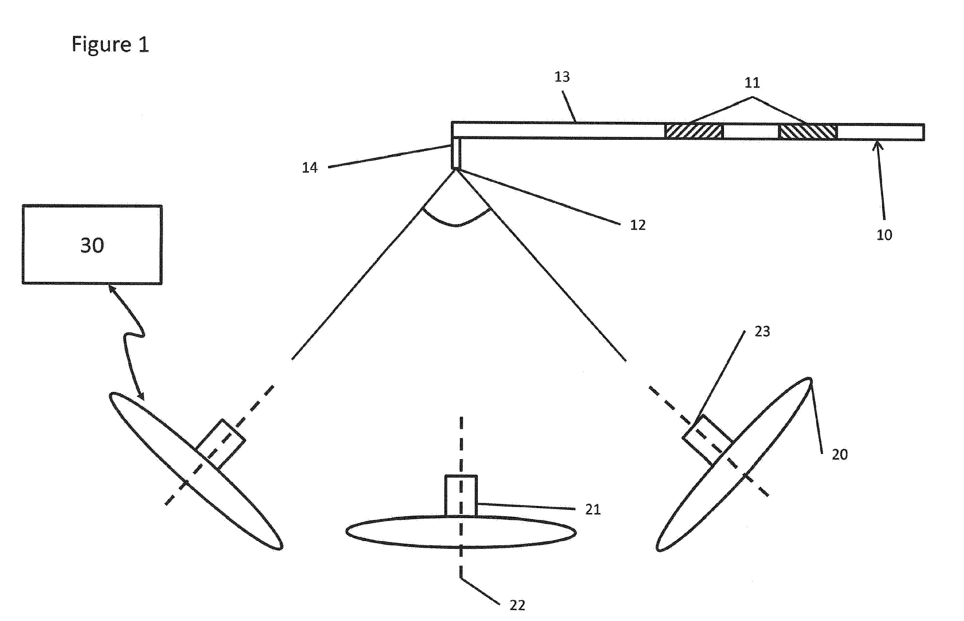

As has been discussed above, the present disclosure relates to a surgical guidance system 1 which comprises a probe 10 and targeter 20. Fig. 1 shows a general representation of the probe 10 and targeter 20. As mentioned there are certain surgical techniques and procedures, for example Transcoracoid-Transclavicular Drilling or Retrograde Drilling of Osteochondritis Dissecans Lesions, wherein it is preferable to drill through a portion of body tissue to a desired site within the body of a patient from a side where the desired end of the drill site cannot be seen. In these situations it is clear that, for example, if the surgeon desires to drill through a bone of a patient to reach the cartilage in a joint, whilst leaving the cartilage generally undamaged by the drilling process, imaging techniques are required to ensure that the drill has both the correct angle and exit point on the bone. Furthermore, it is important to ensure that the drill does not extend too far past the bone into the tissue which is to be protected, and thus in general the precise point as to where the end of the drilled hole should exit the bone, or other body tissue, is desired.

In the system shown in the figures, the targeter 20 may be used to locate the end of the probe 12. During a surgical procedure, the surgeon will initially place the probe 10 such that the end of the probe 12 is located at the desired end point of the hole to be drilled. The positioning of the probe 10 at such a position, and in particular the end of the probe 12, is less invasive than drilling to this position, or otherwise, and the surgeon can thus ensure that minimal damage to surrounding body tissue ensues from the probe 10 placement. Once the end of the probe 12 is in the desired location of the body, the targeter 20 can be used to locate the end of the probe 12, and thus aid the surgeon in drilling to the desired end point and site within the body.

The targeter 20 is preferably provided with a drilling guide 21, wherein the drilling guide 21 can take any form appropriate for ensuring that a drill will proceed in the direction of the drilling guide 21. One example would simply be a hole through the targeter 20, or even a hole through the targeter 20 and an extended elongated tube through which a bore passes, to ensure that the drill proceeds in the correct angle. As has been shown in Fig. 1, the targeter 20 and drill guide 21 define a drill line 22, wherein the drill line 22 is the line along which the drill will progress and the bore in the body tissue will be made. As is clear from the description of Fig. 1, once the targeter 20 locates the end of the probe 12, it is possible to align the drill line 22 with the end of the probe 12 to ensure that the hole which is to be drilled will proceed in the correct direction and will arrive at the desired end point of the drill bore.

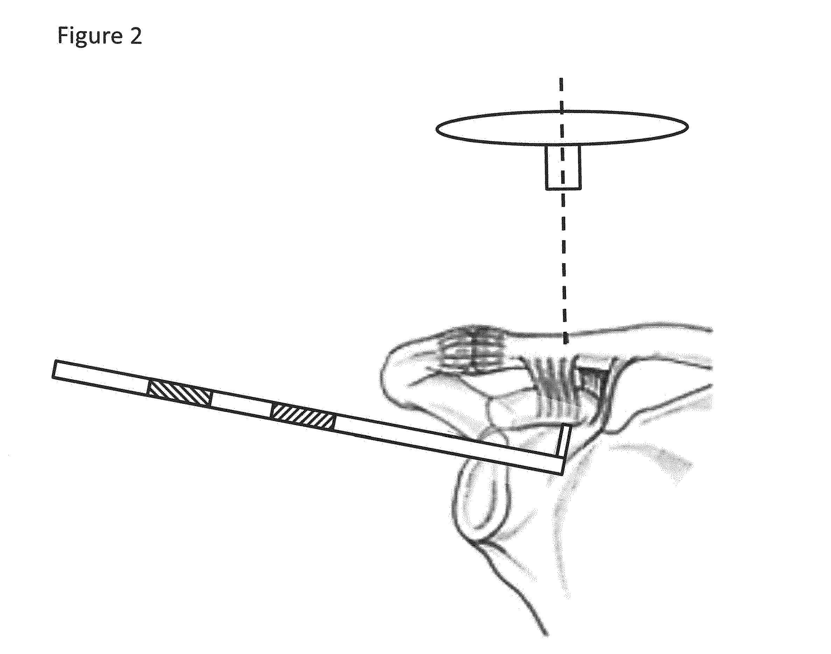

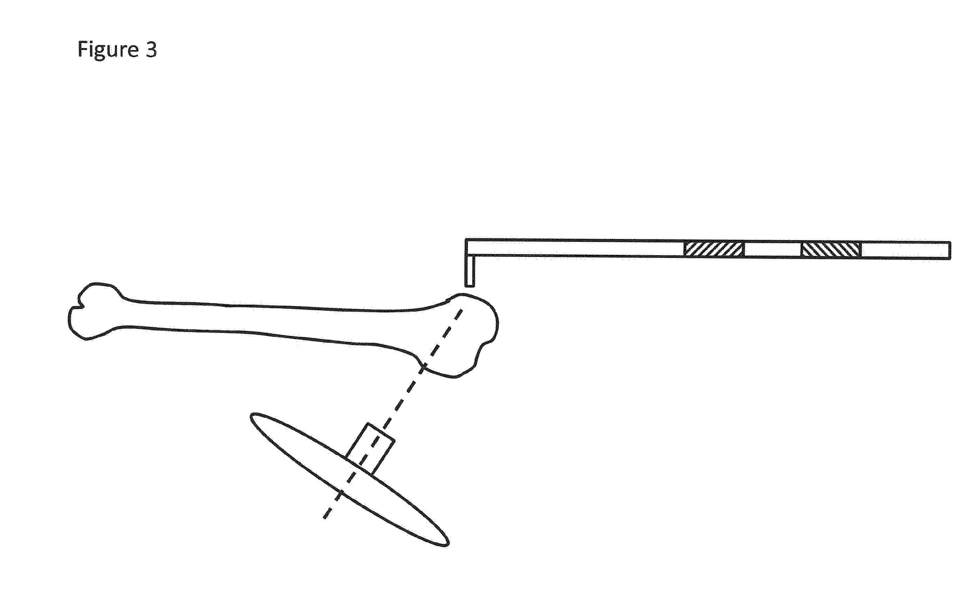

It is also possible by using the system 1 to ensure that the drilled hole does not extend too far. Obviously the end of the probe 12 provides a definite point within the body of the patient, and the targeter 20 can not only guide the angle of the drill line into the direction of the probe 12, but will also be able to determine how far the end of the probe 12 is from the end of the drill guide 23. this ensures that the bore which is drilled in the bone, or other body tissue, will not extend too far and will end exactly at the head of the probe 12. As is clear from this technique, the system allows for great flexibility and accuracy in locating a drill hole through body tissue to an end and desired point - without the need for invasive opening of the joint or other visualisation techniques, as have been discussed in the prior art as being undesirable. Figs. 2 and 3 also show the surgical guidance system 1 in use, wherein the probe 10 can be positioned on one side of a bone, and the targeter 20 can be used to align the drill line 22 such that the drilling will proceed in the appropriate direction and will arrive at the end of the probe 12.

In order to locate the end of the probe 12, the present system 1 preferably incorporates one or more elements 11 on the probe 10 which can be visualised by the targeter 20. In truth, the nature of the elements 11 is in no way limited to a particular feature or structure. The targeter 20 is an electromagnetic targeter, and thus requires only that the position of the one or more elements 11 can be determined by electromagnetic means. The prior art system discussed above to Smith & Nephew, that of the Trigen Sure-Shot System, provides an electromagnetic sensor as the targeter 20, and can be used to target a chip which has been placed into an intramedullary nail. The present system utilises the same concepts as defined in this prior known system, however instead of positioning the chip into an implant, the present system provides the reusable and user positionable probe 10. That is, the known technique uses a targeter 20 as described above to locate a chip which is positioned within an implant which has already been placed within a patient. The prior art therefore, does not allow for the user of the system to pick a desired location in the body as the end point of a drilling line, as it provides an element within an implant which can be sensed by means of an electromagnetic targeter. It is clear from the known system that space requirements for locating an item which can be sensed electromagnetically are of little concern, as the skilled person will realise that positioning an implant within the body of a patient is already a rather invasive technique, and most implants are of a sufficient size to allow for a chip, or other item which can be sensed by the electromagnetic targeter 20, to be positioned therein.

The present case, by contrast, requires that the minimum invasion of the patient's body be made in order to position the end of the probe 12 at the desired end site of the drilling hole. The surgeon would usually use an orthoscopic technique to view the joint or other body part to which a hole must be drilled, and further would require or desire to use a small probe 10 for positioning the end of the probe 12 at the desired end site of the hole. To this end, it is quite clear that the end of the probe 12 - and indeed most of the probe 10 itself, should desirably be of as small a size as possible, thus ensuring that the minimum disruption to the patient's body at the side not being drilled is made. In some surgical techniques it is preferred not to interfere with the other side of the drill line as much as possible, and thus the use of large or bulky probes 10 to locate the end of the drilled hole is undesirable.

To this end, and as can be seen in the figures, the probe 10 may be provided with one or more elements 11 at a position away from the end of the probe 12. By avoiding positioning the elements 11 which can be sensed by the targeter 20 at the end of the probe 12, the end of the probe can be made into a very small and readily positionable point, such that minimum invasion and damage to surrounding tissue occurs. Obviously, however, by providing a very small end to the probe 12 to avoid damage, it is not possible for the targeter 20 to focus and obtain accurate readings of the end 12, as it is also very difficult to put a sufficiently detectable element 11 at the end of the very small probe 12. Furthermore, even if it were possible to put a small element 11 which can be detected by the targeter 20 at the end of the probe 12, in many situations the body tissue, and the like, between the targeter 20 and the element 11 at the end of the probe 12, would lead to significant interference with the detection of the element 11, thus reducing the accuracy of the guidance system 1.

As can be seen in the figures, the newly described system 1 proposes the use of one or more elements 11 away from the distal end of the probe 12. By positioning the elements 11 along the shaft of the probe 13, it is possible to both make the end of the probe 12 a very small point, and also ensure that the targeter 20 can properly visualise the elements 11 and take an appropriate reading therefrom. It will be noted that in the figures two elements 11 are positioned along the shaft 13 of the probe 10, and that these elements 11 are positioned away from the end of the probe 12. As can be seen in Fig. 2, the two elements 11 are positioned a sufficient distance away from the end of the probe 12 such that the two elements 11 are not placed within the body of the patient when the probe 10 is located with the end of the probe 12 at the desired drilling point. In such a situation, it is clear that the body tissue of the patient does not lie between the targeter 20 and the elements 11, thus ensuring that the position of the elements 11 can be properly detected and monitored by means of the targeter 20. This greatly improves the accuracy of the system, whilst also ensuring that a small end of the probe 12 can be used to minimise damage to the patient, when in use.

It will be understood that only one element 11 is strictly necessary in this system, however to improve the accuracy significantly two or more elements 11 can be provided. By positioning the elements 11 along the shaft of the probe 13, it is possible to detect the position of the two or more elements 11. If the two or more elements are measured by the targeter 20, these two points provide a clear direction of the probe 10 in three dimensional space. In order to define a single two dimensional line in a three dimensional space it is necessary to have at least two points, hence the advantage of using two, or more, elements 11 on the probe 10. When the position of the two elements 11 has been determined, it is possible then to determine the precise location of the end of the probe 12 by extrapolating along the known line. As will be seen from the figures, the probe 10 may be provided with a hook 14 as the end of the probe 12, wherein the hook 14 can be used in order to also define both the end of the probe 12 and in some cases also a further extension of the drilling line 22. The positioning of a hook 14 at the end of the probe 10 is not problematic, as the system 1 can also determine the offset from the elements 11 of the end of the probe 12.

In this case a control system 30 shown schematically in some form of communication with the targeter 20 or the like in the figures, is provided which is in communication with the targeter 20. The targeter 20 will take measurements and will locate the positions of the one or more elements 11, and can thus then provide this information to the control system 30. The control system 30 then takes the information as to the position of the elements 11, and can calculate the location of the end of the probe 12. The control system 30 is obviously precalibrated to determine the precise location of the end of the probe 12 from the measurement of the position of the one or more elements 11, even when the probe 10 is provided with a hook 14. To this end, the measurements taken by the targeter 20 are transferred to the control system 30 this can calculate the relative position of the end of the probe 12 with regard to the end of the drill guide 23. Not only is the control system 30 so designed to calculate the relative positions of the end of the probe 12 and the end of the drill guide 23, but it can also take account of the extension of the drill line 22 with respect to the head of the probe 12.

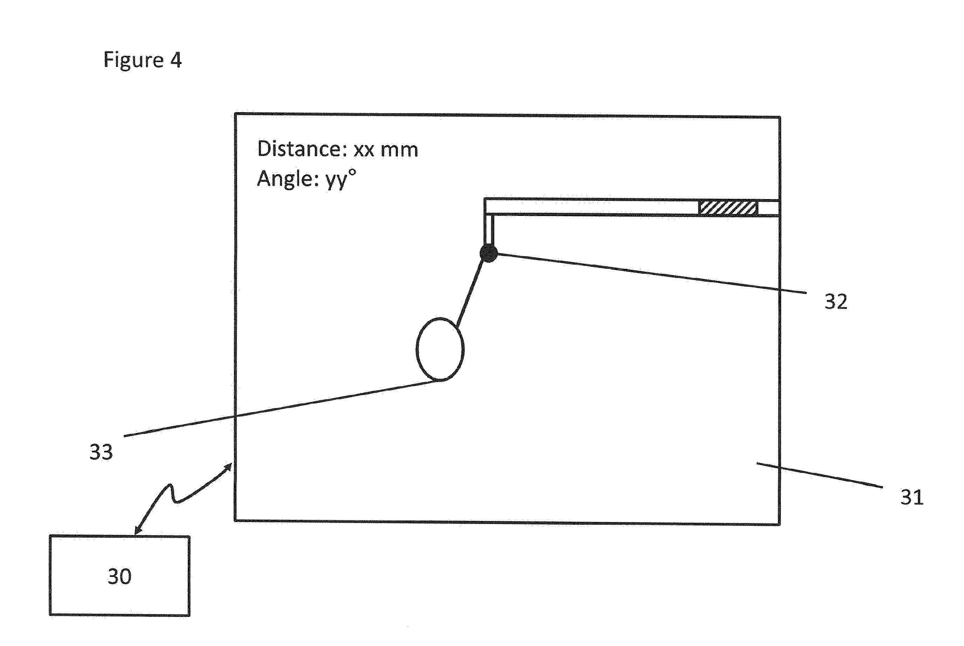

In order to allow the surgeon to properly align the targeter 20 such that the drill line 22 will properly overlap with the end of the probe 12, it is clear that the targeter 20 will need to be moved in order to align the drill guide 21 appropriately. The control system remains in contact with the targeter 20 and thus obtains real time information of the relative locations of the head of the probe 12, the end of the drill guide 23 and the drill line 22. By showing the relationship of each of these, for example on a television or computer screen (although there is no specific restriction as to the nature of the representation) the surgeon can obviously move the targeter 20 until the drill line 22 overlaps with the end of the probe 12. Furthermore, the control system 30 can indicate the distance between the end of the drill guide 23 and the end of the probe 12, along the drill line 22, such that the surgeon knows exactly how far to drill into the patient to reach the end of the probe 12. Furthermore, the system is also adapted to be able to highlight the relative angle between the drill line 22 and the end of the probe 12, and show this on the image. This would allow certain procedures to also ensure that the drill line 22 will not only overlap with the end of the probe 12, but will also be at a specific angle - which can be advantageous in certain surgical procedures. The system will thus provide a real time image indicating both the end of the probe 12 as well as the location of the end of the drill guide 23 and the direction of the drill line 22; this will allow the surgeon to move the targeter 20 and align the drill line 22 with the end of the probe 12. Such a system is schematically shown in Fig. 4, wherein the end of the probe 12 is highlighted by the dot 32, the circle 33 represents a physical location of the end of the drill guide 23 in relation to the end of the probe 12, and the line represents the drill line 22. Of course, different representations could be shown, for example the circle 33 shown in Fig. 4 could provide an image showing purely the direction along which the drill line 22 extends, such that movement of the targeter 20 will also move the circle 33, and this need only then be overlapped with the position of the end of the probe 12 to ensure that the drill will proceed in the correct direction. The nature of the visual representation 31 is not relevant for the teachings of the present disclosure, and any useful graphical, or even numerical, representation given in real time to the surgeon is sufficient for allowing the targeter 20 to be properly aligned with the end of the probe 12.

As will be appreciated from the above, the guidance system 1 would be greatly improved in usability when the visual representation of the end of the drill guide 23 as well as the drill line 22 and the end of the probe 12 aligns with the "real world" orientation of the probe 10 and targeter 20. In the current text, the term "real world" will always be used to indicate the physical orientations and positions of all elements of the guidance system 1 in and around the user of the system and patient. The real world orientations are those which the user of the system perceives and in essence lives in, as opposed to the graphical representation 31 on the display device of the guidance system 1.

As will be understood, the location of the end of the probe 12 can be determined from sensing of the one or more elements 11. In particular if multiple elements 11 are provided, then the directional line of the probe 10 can be understood by the control system 30, and the location of the end of the probe 12 can be derived therefrom. A possible way of determining the actual direction of the probe 10 is to provide the elements 11 with a different nature which can be sensed by the control system 30. For example, if each of the multiple elements 11 were to be different - for example a coil which can be sensed by the EM sensor of the targeter 20, or an RFID tag, or a denser portion of the shaft 13, or the like - it is possible for the actual direction of the probe 10 with respect to the targeter 20 to be determined. Once the control system 30 knows the direction of the probe 10, and in particular the direction of the shaft 13, the position of the end of the probe 12 can be located with respect to the targeter 20. In particular, the position of the end of the probe 12 can be determined with respect to the end of the drill guide 23 and the drill line 22.

A problem exists in this system, however, in translating the real world orientation of the probe 10 and targeter 20 onto the visual representation 31 for the surgeon. Unless the control system 30 has knowledge of the actual, real world, orientation of the probe 10 with respect to the user - and not just the targeter 20 - the graphical representation 31 will not have internal directions which match the real world directions for the user. This therefore means that when the targeter 20 is moved to the left by the user, unless there is a correlation between the real world direction for the user and the graphical direction 31 of the control system 30, there is little chance that the movement shown on the screen will match the real world movement of the targeter 20. It is undesirable to have a misalignment between the real world directions and the internal directions of the guidance system 1, as this reduces the intuitive nature of the entire guidance system 1. To this end, it is necessary to determine a relationship between the onscreen directions and the real world directions associated with the probe 10 and targeter 20.

In general, a simple technique would be to always ensure that the probe 10 extends in a known direction to the control system 30, such that once the location of the one or more elements 11, and thus the end of the probe 12, have been determined, the movement of the targeter 20 is also properly aligned with the image on the screen. Unfortunately, it is very rarely possible to ensure that the probe 10 can be introduced into the drill site and body in a known orientation, consider that this must be done in what is essentially a full three dimensional environment and the precise angle of the probe 10 must align with that stored in the control system 30. As such, the present guidance system 1 proposes some form of calibration to ensure that the real world directions of the probe 10 and targeter 20 are aligned with the visualisation directions as determined by the control system 30. By beginning a surgical procedure the user can thus calibrate the known position of the probe 10 with the targeter 20, and ensure that the control system 30 has a clear understanding of the actual physical orientation in the real world of the probe 10 and can thus ensure that relative movements of the targeter 20 will then be shown accurately on the visual representation 31.

A number of mechanisms exist for properly determining the real world orientation of the probe 10, and one will be described here. It is clear, however, that what is required is a possibility of calibrating the physical real world location and orientation of the probe 10 in the control system 30, such that real world movement of the targeter leads to the same movement on the visual representation 31. As such, the disclosed and proposed calibration should not be considered as only the solution to this issue, and the skilled person will readily be able to derive other mechanisms of determining the real world orientation of the probe 10.

As a described example, the user could begin the calibration routine by means of some triggering signal. After the triggering signal is received by the control system 30, the control system 30 can then guide the user of the system through a series of motions of either the probe 10 or targeter 20, to ensure that the location of the probe 10 in the real world can be monitored by the control system 30 such that later relative movements between the targeter 20 and the probe 10 can be co-ordinated between the real world and the visual representation 31. It is generally easier to provide the probe 10 in a fixed orientation and move the targeter 20 with respect thereto, however the skilled person will realise that the exact opposite will also allow the system 30 to determine the orientation of the probe 10.

The nature of the triggering signal is not really limited to any single technique. It is desirable on the part of the user to avoid having to interact with anything other than the probe 10 and targeter 20, and thus a preferred technique is generally one which requires the user not to have to interact with the control system 30 directly or physically. For example, the end of the drill guide 23 could be brought into contact or very near contact with the end of the probe 12, this can be registered by the control system 30 as a triggering signal which will then lead the control system 30 to begin a calibration routine. Further possibilities are that a known motion of the targeter 20 could be used in relation to the probe 10 in order to begin the calibration routine. Such a motion of the targeter 20 would be advantageously rather large and unlikely to occur in any surgical situation, so as to avoid inadvertent starting of the calibration routine during an actual surgical procedure. Further, the triggering signal could be the physical removal of the targeter 20 away from the probe 10, such that the field of view of the targeter 20 no longer comprises the probe 10 at all, and maintaining the targeter 20 away from the probe 10 for a sufficiently long time to ensure resetting of the system. As is clear from the above, any form of motion between the probe and targeter 20 can be used to start the calibration routine, and thus the system is not really limited to any of the possibilities.

Once the calibration routine has started, the simplest mechanism of determining the real world orientation of the probe 10 by the control system 30, is to lead the user of the system through a series of defined motions of the targeter 20 in the real world. For example, moving the targeter 20 to the left; followed by to the right; followed by up; followed by down, will lead the control system 30 to fully understand the physical orientation of the probe 10 in the real world, and thus the probe 10 orientation can be stored and the visual representation 31 between the motion of the targeter 20 with respect to the probe 10 can be coordinated for easy use by the surgeon. Other motions of the targeter 20 can also be used to indicate the location of the probe 10, and in fact the probe 10 can be moved instead of moving the targeter 20. The actual mechanism by which the control system 30 determines the orientation of the probe 10 is, as stressed a second time, not limited, and it is the provision of the calibration routine in order for the control system 30 to learn the real world orientation of the probe 10 that is important.

Once the orientation of the probe 10 is known to the control system 30, movement of the targeter 20 with respect to the probe 10 will then match between the real world and the visual representation 31, thus improving the intuitive nature of the system for the user. Given that any surgical procedure needs to be achieved with the greatest accuracy in the shortest amount of time, to avoid unwanted infection to the patient for one, this improvement to the intuitiveness is greatly appreciated. Further, by means of this simple calibration routine, accuracy of the surgery will also be increased as the surgeon can focus on ensuring that the drill line 22 and length of the drilled hole will properly match such that the end of the probe 12 is met.

It will be understood from the above that after the calibration routine has been followed, the orientation of the probe 10 in the real world should probably not change too much from that of the targeter 20. In order to allow the surgeon to generally determine the real world orientation of the probe 10 and store this in the control system 30, and then have freedom to position the probe 10 at the desired surgical site, a preferable further step to the calibration method is to allow for the system to monitor the movement of the probe 10 and update the stored probe 10 orientation. As can be understood, once the orientation of the probe 10 is determined at the end of the calibration routine, the targeter 20 could be maintained in its known orientation and monitor the motion of the probe 10 as this is placed into the desired surgical site. After either a predetermined length of time, or length of inactivity of the motion of the probe 10 with respect to the targeter 20, or indeed any other predetermined further signal, the control system 30 could stop updating the orientation of the probe 10 and then allow for the relative movement between the end of the probe 12 and the targeter 20 to be understood as motion of the targeter 20 to align the drill line 22 to the end of the probe 12. That is, after a certain amount of learning time, the control system 30 assumes that the probe 10 is in location such that the end of the probe 12 now marks the desired end point of the drill, such that the motion of the targeter 20 is then treated as a targeting motion, rather than a calibration motion. In this manner, even motion of the probe 10 after calibration will be updated as the orientation of the probe in the system, further increasing the accuracy of the system 1.

A further advantage of the second probe 10 orientation storage, is that should the surgeon need to dramatically change the orientation of the probe 10 in the real world as a result of trying to get to the site at the end of the drill hole, it will not be necessary to go through the calibration routine a second time. Again this improves the interaction of the surgeon with the display and greatly improves the accuracy of using the guidance system 1. Furthermore, the speed of using the system 1 is increased which again reduces the length of a surgical procedure which is always desirable.

In the above description and figures it is clear that the system is shown with a combined targeter 20 and drill guide 21, however this is for convenience only - at all points the skilled person will appreciate that the targeter 20 could be separate from the drill guide 21. Indeed, in some surgical techniques it is actually preferable for the drill guide 21 to be a separate item and not part of the targeter 20. In such cases the surgeon could locate the separate drill guide 21 in the desired orientation and position - based on the measurements made by the targeter 20 for locating the end of the probe 12. An assistant could hold the targeter 20 behind the separate drill guide 21 along the same line as the drill line 22 of the drill guide 21, so as to allow alignment of the drill guide 21 with the end of the probe 12. Another possibility would be to provide the separate drill guide 21 with elements, in an analogous manner to those positioned on the probe 10, such that the control system 30 could also measure the line location and direction of the drill guide 21 in relation to the end of the probe 12, so as to allow the targeter 20 to be generally located and sensing the surgical site, wherein the control system 30 takes the measurements from both the probe 10 and the separate drill guide 21 and provide an appropriate image for the surgeon on the visual representation 31.

As will be understood above, a number of features and aspects relating to the control system 30, probe 10 and targeter 20 have been described. From this description the skilled person understands that many elements can be derived as both individual elements of the system and elements in combination with other features. No clear description of element combinations should be understood from the above disclosure over and above what would be logical in order to allow for determination of the end of the drill hole, and appropriate calibration of the control system 30 to the real world direction. To this end, the above should be considered as a description of aspects and concepts, which when logical can be combined to generate an appropriately functioning system. The claims attached to this disclosure provide a description of the key aspects of the disclosure.

## Claims

1. A surgical guidance system (1) for orthoscopic drilling comprising:
a probe (10), the end (12) of which is suitable for locating on the emergence site of a hole to be drilled through body tissue,
a targeter (20)comprising one or more sensors for locating the position of the probe (10),
a drilling guide (21),
a control system (30) in communication with the targeter (20) for collating the position of the probe (10) measured by the targeter (10) and providing a real-time visual representation (31) of the relationship between the direction of the drilling guide (21) and the end of the probe (12) to allow the user to align the drilling guide (21) to the end of the probe (12),
wherein the probe (21) comprises an elongate shaft (13) and one or more, preferably two or more, elements (11) located at physically separated positions along the shaft (13), and wherein further the elements (11) can be detected by the targeter (20) and are each positioned at locations removed from the end of the probe (12).

2. The system (1) of claim 1 wherein the control system (30) is adapted to determine from the measurements of the locations of the elements (11), as made by the targeter (20), the location of the end of the probe (12), and thus the exit point for the hole to be drilled and/or the angle between the drilling guide drill line (22) and the end of the probe (12).

3. The system (1) of any one of the previous claims wherein the control system (30) is adapted to provide an image on a screen, or other visual representation (31) indicating the location of the end of the probe, as well as an indication of the direction in which the drilling guide (21) is pointing, in particular the drill line (22) of the drilling guide (21), in relation to the end of the probe (12).

4. The system (1) of any one of the previous claims, in particular claim 3, wherein the control system (30) is adapted to provide the image showing the relationship between the direction of the drilling guide (21) and the end of the probe (12) in correlation with the "real world" directions of the probe (10), the drilling guide (21) and targeter (20), such that movement of the targeter (20) and/or drilling guide (21) leads to a corresponding movement of the image showing the direction of the drilling guide (21) on the screen, so as to improve the intuitive use of the instrument for the user.

5. The system (1) of any one of the previous claims wherein the control system (30) is adapted to perform calibration of the real world direction of the probe (10) and movement of the targeter (20), thus allowing the probe (10) to be oriented in any real world orientation with respect to the targeter (20).

6. The system (1) of any one of the previous claims wherein the control system (30) is adapted to begin a calibration routine to determine the real world positional relationship between the probe (10) and the targeter (20) after a triggering act is performed by the user.

7. The system (1) of any one of the previous claims wherein the triggering act is any one or more of:
a) bringing the end of the targeter (23) into close proximity or contact with the end of the probe (12),
b) performing a certain, predefined, set of motions of the targeter (20) near the probe (10),
c) actuating a switch on the targeter (20) or probe (10),
d) moving the targeter (20) so that the probe (10) is out of its field of view for more than a predetermined time.

8. The system (1) of any one of the previous claims wherein for a period of time after the calibration routine has been successful performed, the control system (30) is adapted to monitor the relative movement of the probe (10) with respect to the targeter (20) and to update the calibration direction should orientation changes of the probe (10) occur.

9. The system (1) of any one of the previous claims wherein the distance between the one or more elements (11) and the end of the probe (12) is larger than the body part into which the end of the probe (12) will be positioned.

10. The system (1) of any one of the previous claims wherein the one or more elements (11) are one of coils, magnets, RFIDs, denser portions of the shaft, or any other electromagnetically discernible elements and preferably the targeter (20) comprises an electromagnetic sensor for imaging the one or more elements (11) on the shaft (13).

11. The system (1) of any one of the previous claims wherein two or more elements (11) are provided and the nature of each element is preferably different, wherein this nature can be embodied by each of the elements being different, or having a different and discernible orientation, or having a different associated code.

12. The system (1) of any one of the previous claims, wherein the targeter (20) comprises a drilling guide (21) to form a combined targeter-drilling guide unit.

13. A method for calibrating the surgical guidance system (1) of any one of the previous claims, the method comprising:
performing a triggering step so as to reset any already stored real world orientation of the probe (10) in the controls system (30),
providing visible instructions to the user via the visual representation (31) of the system for moving either one of the targeter (20) or probe (10) with respect to the other of the probe (10) or targeter (20),
monitoring the relative position of the probe (10) and targeter (20) in the control system (30),
determining the real world orientation of the probe (10) from the relative movement between the probe (10) and the targeter (20) and storing this real world orientation.

14. The method according to claim (13), wherein once the control system (30) has determined and stored the real world orientation of the probe (10), the control system (30) provides real time images wherein the motion of the targeter (20) and drilling guide (21) in the real world are properly calibrated with the visual representation (31), such that movement of the targeter (10) and/or drilling guide (21) in the real world provides the same movement on the visual representation (31).

15. The method according to either one of claims (13) or (14), wherein after the step of determining the real world orientation and storing this, the method continues by:
monitoring the relative motion between the targeter (20) and probe (10), wherein changes to the orientation of the probe (10) are registered and after a set time and/or a period of time where no change in orientation of the probe (10) occurs the new orientation of the probe (10) is determined to be the actual real world orientation and is stored.
